# FASCICULE DE BREVET EUROPEEN

(11) **EP 0 848 752 B1**
(45) Date de publication et mention de la délivrance du brevet: **04.06.2003**
(21) Numéro de dépôt: 96924954.9
(22) Date de dépôt: 08.07.1996
(51) Int. Cl.: C12N 7/02, A61K 39/12, A61K 39/145, A61K 39/205

(54) **PROCEDE DE PURIFICATION DE VIRUS PAR CHROMATOGRAPHIE**
VERFAHREN ZUR REINIGUNG VON VIREN MIT CHROMATOGRAPHIE
METHOD FOR PURIFYING VIRUSES BY CHROMATOGRAPHY

(30) Priorité: 10.08.1995 FR 9509851
(43) Date de publication de la demande: 24.06.1998
(73) Titulaire: Aventis Pasteur, 69367 Lyon Cedex 07 (FR)
(72) Inventeur: FANGET, Bernard, F-69210 Saint-Germain-sur-l'Arbresle (FR); FRANCON, Alain, F-69690 Bessenay (FR)
(74) Mandataire: Kerneis, Danièle
(86) Numéro de dépôt international: FR9601064
(87) Numéro de publication internationale: WO97006243

(56) Documents cités:
- WO-A-95/01797
- FR-A- 1 441 767
- US-A- 4 664 912
- CHEMICAL ABSTRACTS, vol. 51, no. 1, 10 Janvier 1957 Columbus, Ohio, US; abstract no. 530f, H.D. MATHEKA ET AL.: "ION EXCHANGE FOR VIRUS PREPARATIONS. I. INFLUENCE OF THE ION-EXCHANGE PROPERTIES ON THE ADSORPTION OF INFLUENZA VIRUS." XP002000499 & Z. NATURFORSCH., vol. 11b, 1956, pages 187-193,
- CHEMICAL ABSTRACTS, vol. 51, no. 1, 10 Janvier 1957 Columbus, Ohio, US; abstract no. 530g, H.D. MATHEKA ET AL.: "ION EXCHANGE FOR VIRUS PREPARATIONS. II. FRACTIONATION OF INFLUENZA VIRUS" XP002000500 & Z. NATURFORSCH., vol. 11b, 1956, pages 193-199,

## Description

L'invention concerne le domaine de la purification de virus, et plus particulièrement la purification de virus obtenus par cultures sur lignées cellulaires.

Les récoltes de virus obtenus à partir de cultures sur lignées cellulaires telles que les cellules Vero, contiennent non seulement les virus désirés mais aussi des protéines et de l'ADN provenant des cellules de culture. Or, lorsque les virus sont destinés à certains usages, tels que la fabrication de vaccins, il est indispensable qu'ils soient le plus purs possible. En effet, il existe des normes limitant à 100.10⁻¹²g/dose vaccinale la quantité maximale autorisée d'ADN cellulaire dans les vaccins comprenant des produits obtenus à partir de lignées cellulaires continues et hétéroploïdes.

On connaît dans l'art antérieur des procédés de purification de virus. Ainsi, le brevet US 4 664 912 divulgue notamment un procédé de purification de virus de la rage par centrifugation de zone dans un gradient de sucrose. Un tel procédé présente cependant l'inconvénient d'être difficilement automatisable ; en outre, il nécessite une étape d'inactivation préalable qui peut entraîner entre le virus et l'ADN cellulaire des interactions rendant ensuite plus difficile l'étape d'élimination de cet ADN.

Cette référence divulgue également un procédé de purification associant une étape de filtration sur gel et une étape de chromatographie échangeuse d'ions. Un tel procédé de purification ne permet cependant pas d'obtenir une élimination maximale de l'ADN cellulaire.

Un but de l'invention est donc de proposer un nouveau procédé de purification de virus à très haut rendement et facilement automatisable.

Un autre but de l'invention est de proposer un procédé de purification permettant d'obtenir des virus entiers, non dégradés.

Pour atteindre ces buts, l'invention a pour objet un procédé de purification de virus obtenus à partir d'une culture sur lignée cellulaire, consistant à séparer par chromatographie échangeuse d'ions les virus des protéines et ADN cellulaires provenant de la culture, caractérisé en ce qu'il comprend au moins une étape de chromatographie échangeuse d'anions et une étape de chromatographie échangeuse de cations.

Selon une caractéristique particulière du procédé selon l'invention, l'étape de chromatographie échangeuse de cations est effectuée après l'étape de chromatographie échangeuse d'anions. On obtient ainsi des rendements d'épuration optimisés par rapport à l'inversion de l'ordre des étapes.

Selon un mode particulier de réalisation, le procédé selon l'invention comprend en outre une étape de chromatographie d'affinité par chélation métallique. Ainsi, les quantités d'ADN résiduel sont vraiment réduites au maximum.

Selon un autre mode préféré de réalisation, le procédé selon l'invention consiste en outre à effectuer toutes les étapes de chromatographie à la même valeur de pH. Il est ainsi possible d'automatiser facilement le procédé et d'en réduire notablement les coûts, tout en maintenant son efficacité.

L'invention sera mieux comprise à la lecture de la description détaillée qui va suivre.

Les virus à purifier selon le procédé de l'invention sont des virus obtenus grâce à des lignées cellulaires, notamment des lignées cellulaires continues et hétéroploïdes et qui sont donc susceptibles d'être associés à des ADN cellulaires. Il peut s'agir notamment de virus rabiques, de virus de l'encéphalite japonaise ou encore de virus de la grippe. Ces virus à purifier peuvent avoir été obtenus par culture sur cellules Vero sur microsupports. Il s'agit par exemple du virus rabique obtenu par culture ainsi que cela est décrit dans le brevet US 4 664 912.

La récolte de virus est filtrée puis traitée, selon l'invention, par chromatographie échangeuse d'anions et par chromatographie échangeuse de cations.

La chromatographie échangeuse d'anions peut être une chromatographie échangeuse d'anions faibles réalisée par exemple au moyen de gel comportant le radical diéthylaminoéthyl : gel DEAE Spherodex commercialisé par SEPRACOR, gel DEAE Sepharose commercialisé par PHARMACIA, gel EMD DEAE commercialisé par E. MERCK. On préfère, bien que cela ne soit pas habituel pour l'élimination d'acides nucléiques, utiliser un échangeur d'anions forts tels que un des gels suivants : gel High Q commercialisé par BIO-RAD, gel Q Sepharose commercialisé par PHARMACIA, gel EMD TMAE commercialisé par E. MERCK.

Tous ces gels peuvent être utilisés en tampon TRIS ou en tampon phosphate. De façon avantageuse on utilise un gel qui permet une grande disponibilité du groupement cationique, tel que cela est le cas dans l'EMD TMAE de E. MERCK qui possède des chaînes de 15 à 25 unités de monomère suivant :

CH₂=CHCONH(CH₂)₂N⁺(CH₃)₃

De même, selon le procédé de l'invention, l'étape de chromatographie échangeuse de cations est de préférence réalisée au moyen d'un gel permettant une grande accessibilité du groupement anionique, tel que cela est le cas dans l'EMD SO₃ commercialité par E. MERCK, qui possède des chaînes de 15 à 25 unités du monomère suivant :

CH₂=CHCONH-C(CH₃)₂CH₂SO₃⁻

Ce gel peut également être équilibré au moyen de tampon TRIS ou de tampon phosphate.

Selon un mode préféré de l'invention, on procède à la chromatographie échangeuse d'anions préalablement à la chromatographie échangeuse de cations, l'éluat de la première chromatographie étant, après dilution éventuelle, introduit directement dans la seconde colonne de chromatographie. Cet ordre d'opération permet d'obtenir une optimisation de l'élimination de l'ADN cellulaire.

La suspension de virus obtenue par élution de la colonne de chromatographie échangeuse de cations présente un degré de pureté déjà satisfaisant vis-à-vis des normes établies pour l'utilisation des virus dans la fabrication des vaccins.

Cependant, selon un mode préféré de l'invention, on adjoint au procédé de purification décrit ci-dessus une étape supplémentaire de chromatographie d'affinité par chélation métallique, en utilisant par exemple l'ion Ca²⁺ comme ion métallique. Cette chromatographie peut être réalisée en utilisant comme support de chélation un gel d'agarose, tel que le gel chelating Sepharose® FF commercialisé par PHARMACIA, qui possède la structure suivante :

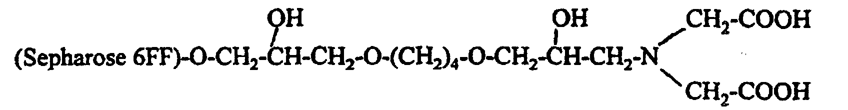

Selon le procédé de l'invention, cette étape de chélation est réalisée en ligne avec les 2 étapes précédentes de chromatographie échangeuse d'ions, l'éluat de l'échangeur de cations étant introduit directement dans la colonne de chromatographie de chélation.

Lors de cette étape, l'ADN cellulaire reste fixé alors que le virus traverse le gel sans être retenu.

Cette étape additionnelle permet de fixer encore au moins la moitié de l'ADN résiduel qui est certes déjà en très faible quantité mais qui est en revanche, à ce stade, très difficile à éliminer.

La suspension de virus ainsi purifiée est diluée par un stabilisant puis inactivée selon toute méthode connue dans l'art antérieur, par exemple à la βpropiolactone, avant d'être concentrée et lyophilisée, pour être ensuite mélangée aux autres récoltes issues de la même culture de cellules VERO et qui vont constituer un même lot de fabrication.

Les vaccins fabriqués à partir de tels virus ont été injectés à l'homme et ont été très bien tolérés. On a, en outre, vérifié leur activité protectrice chez l'animal grâce à un test effectué chez la souris dont les résultats ont été positifs.

De façon surprenante et très avantageuse, selon l'invention, il est possible de réaliser l'ensemble de la purification à température ambiante en ligne et toujours au même pH, ce qui rend le procédé simple, facilement automatisable et sûr ; il est notamment possible de réaliser l'ensemble du procédé dans de bonnes conditions de stérilité.

Grâce à ce procédé de purification, on obtient des virus entiers, non dégradés dont les spicules sont visibles en microscopie électronique, avec un très haut degré de pureté.

### Exemple 1

On prépare une colonne de chromatographie échangeuse d'anions en introduisant dans une colonne du gel EMD TMAE commercialisé par E. MERCK dans laquelle on fait tout d'abord passer de la soude NaOH 1M dans un but de désinfection. La colonne est ensuite équilibrée en tampon TRIS 20mM à pH 7,5.

On prépare une colonne de chromatographie échangeuse de cations en introduisant dans une colonne du gel EMS SO₃ commercialisé par E. MERCK, dans laquelle on fait également passer de la soude 1M puis du tampon TRIS 20mM à pH 7,5.

On prépare une colonne de chromatographie d'affinité par chélation métallique en introduisant dans une colonne du gel chelating Sepharose® FF commercialisé par PHARMACIA dans laquelle on fait passer de la soude 1M puis de l'eau, avant de la charger en CaCl₂ à 3g/l ; on rince ensuite à l'eau puis on fait passer du tampon TRIS 20mM NaCl 0,5M à pH 7,5.

### Exemple 2

On filtre une récolte de virus rabiques cultivés sur cellules VERO grâce à une membrane dont le seuil de coupure est de 0,65µm.puis une membrane dont le seuil de coupure est de 0,45 µm.

Cette récolte est constituée par une suspension de virus dans du milieu de culture comprenant également de l'ADN cellulaire ainsi que des protéines provenant à la fois des cellules VERO et du milieu de culture. La récolte brute filtrée est passée au travers de la colonne de chromatographie échangeuse d'anions préparée selon ce qui est décrit à l'exemple 1. La partie non fixée, contenant des protéines et de l'ADN est recueillie en sortie de la colonne à des fins de dosage. On rince la colonne par du tampon TRIS 20mM NaCl 0.1 M à pH 7,5 puis on élue les virus qui se sont fixés en faisant passer dans la colonne du tampon TRIS 20 mM NaCl 0,4 M à pH 7,5 jusqu'à ce que le pic caractéristique des virus soit passé. L'éluat de la première colonne est dilué au 1/4 en ligne par du tampon TRIS 20mM à pH 7,5 avant d'être introduit dans la colonne de chromatographie échangeuse de cations préparée selon l'exemple 1.

La partie non fixée de cette seconde colonne est recueillie à des fins de dosage. Lorsque tout l'éluat de la première colonne contenant du virus est passé au travers de la seconde colonne, on la rince avec du tampon TRIS 20mM Nacl 0,1 M à pH 7,5 puis on procède à l'élution des virus fixés en faisant passer du tampon TRIS 20mM NaCl 0,5M à pH 7,5 jusqu'à ce que le pic caractéristique des virus soit passé.

L'éluat de cette seconde colonne est introduit directement, sans dilution, dans la colonne de chromatographie d'affinité par chélation métallique préparée selon l'exemple 1. Cette fois, l'ADN se complexe aux ions Ca⁺⁺ présents dans la colonne alors que le virus traverse la colonne et est recueilli en sortie pour être associé à un stabilisant et inactivé. L'ADN complexé aux ions Ca⁺⁺ est élué par passage dans la colonne d'une solution EDTA 50mM.

Les colonnes de chromatographie échangeuses d'ions sont régénérées par passage de tampon TRIS 20mM NaCl 1M à pH 7,5 et désinfectées par de la soude 1M, avant d'être utilisées pour l'épuration des autres récoltes provenant de la même culture qui vont toutes être réunies pour constituer un seul lot de fabrication.

### Exemple 3

On dose pour chaque récolte, après chaque étape de chromatographie, les quantités de protéines et d'ADN cellulaires épurées.

Les protéines totales sont dosées grâce à la méthode de Lowry et l'ADN total au moyen de l'appareil THRESHOLD de MOLECULAR DEVICE.

Les résultats obtenus en faisant une moyenne des rendements par étapes pour chaque récolte de la culture, exprimés en pourcentage résiduel pour chacune des étapes du procédé d'épuration, sont les suivants :

| **Rendement (%)** | **ADN total** | **Protéines Totales** |
|---|---|---|
| Colonne échangeuse d'anions | 0,02 | 0,056 |
| Colonne échangeuse de cations | 27 | 25 |
| Colonne d'affinité par chélation métallique | 80 | |

Globalement, grâce au procédé d'épuration selon l'invention, il ne reste plus que 0,004% de l'ADN cellulaire et 1,4 % des protéines présents dans les récoltes brutes.

L'épuration réalisée est donc complètement satisfaisante.

On vérifie en outre l'infectivité des virus obtenus à chaque étape, grâce à une mesure de leur titre infectieux en DICC 50 et à une observation au microscope électronique. On remarque que l'intégrité du virus est bien conservée tout au long du procédé.

Lorsque les virus ainsi produits sont utilisés dans la fabrication de vaccins contre la rage, il est possible de réaliser des doses ne contenant pas plus de 30 à 40.10⁻¹²g d'ADN cellulaire par dose, soit une quantité nettement inférieure à la norme qui est de 100.¹⁰⁻¹²g.

## Revendications

1. Procédé de purification de virus obtenus à partir d'une culture cellulaire consistant à séparer les virus des protéines et ADN cellulaires provenant de la culture par chromatographie échangeuse d'ions, **caractérisée en ce qu'**il comprend au moins une étape de chromatographie échangeuse d'anions ainsi qu'une étape de chromatographie échangeuse de cations.

2. Procédé selon la revendication 1, **caractérisé en ce que** les anions sont des anions forts.

3. Procédé selon une des revendications précédentes, **caractérisé en ce que** les cations sont des cations forts.

4. Procédé selon une des revendications précédentes, **caractérisé en ce que** l'échange de cations est réalisé après l'échange d'anions.

5. Procédé selon une des revendications précédentes, **caractérisé en ce qu'**il comprend en outre une étape de chromatographie d'affinité par chélation métallique.

6. Procédé selon la revendication précédente, **caractérisé en ce que** la chélation métallique est réalisée au moyen d'ions calcium.

7. Procédé selon une des revendications précédentes. **caractérisé en ce que** les virus à purifier sont obtenus par cultures sur lignées cellulaires continues et hétéroploïdes.

8. Procédé selon une des revendications précédentes, **caractérisé en ce que** les virus à purifier sont obtenus par cultures sur cellules VERO.

9. Procédé selon une des revendications précédentes, **caractérisé en ce que** les virus à purifier sont des virus rabiques.

10. Procédé selon une des revendications 1 à 9, **caractérisé en ce que** les virus à purifier sont des virus de l'encéphalite japonaise.

11. Procédé selon une des revendications 1 à 9, **caractérisé en ce que** les virus à purifier sont des virus de la grippe.

12. Procédé selon une des revendications précédentes, **caractérisé en ce qu'**il consiste à effectuer toutes les étapes de chromatographie à la même valeur de pH.

13. Procédé selon une des revendications précédentes, **caractérisé en ce qu'**il s'agit d'un procédé en ligne.

14. Procédé selon une des revendications précédentes, **caractérisé en ce qu'**il est réalisé à température ambiante.

15. Procédé selon une des revendications précédentes, **caractérisé en ce qu'**il est réalisé dans des conditions stériles.

## Patentansprüche

1. Verfahren zur Reinigung von Viren, erhalten aus einer Zellkultur, das darin besteht, die Viren von den Proteinen und Zell-DNA, die aus der Kultur stammen, durch lonenaustausch-Chromatographie abzutrennen, **dadurch gekennzeichnet, dass** es wenigstens einen Schritt zur Anionenaustausch-Chromatographie sowie einen Schritt zur Kationenaustausch-Chromatographie umfasst.

2. Verfahren gemäß Anspruch 1, **dadurch gekennzeichnet, dass** die Anionen starke Anionen sind.

3. Verfahren gemäß einem der vorhergehenden Ansprüche, **dadurch gekennzeichnet, dass** die Kationen starke Kationen sind.

4. Verfahren gemäß einem der vorhergehenden Ansprüche, **dadurch gekennzeichnet, dass** der Kationenaustausch nach dem Anionenaustausch durchgeführt wird.

5. Verfahren gemäß einem der vorhergehenden Ansprüche, **dadurch gekennzeichnet, dass** es außerdem einen Schritt zur Affinitätschromatographie durch Metallchelation umfasst.

6. Verfahren gemäß dem vorhergehenden Anspruch, **dadurch gekennzeichnet, dass** die Metallchelation mit Calciumionen durchgeführt wird.

7. Verfahren gemäß einem der vorhergehenden Ansprüche, **dadurch gekennzeichnet, dass** die zu reinigenden Viren durch Kulturen auf permanenten und heteroploiden Zelllinien erhalten werden.

8. Verfahren gemäß einem der vorhergehenden Ansprüche, **dadurch gekennzeichnet, dass** die zu reinigenden Viren durch Kulturen auf VERO-Zellen erhalten werden.

9. Verfahren gemäß einem der vorhergehenden Ansprüche, **dadurch gekennzeichnet, dass** die zu reinigenden Viren Tollwutviren sind.

10. Verfahren gemäß einem der Ansprüche 1 bis 9, **dadurch gekennzeichnet, dass** die zu reinigenden Viren Viren der japanischen Enzephalitis sind.

11. Verfahren gemäß einem der Ansprüche 1 bis 9, **dadurch gekennzeichnet, dass** die zu reinigenden Viren Grippeviren sind.

12. Verfahren gemäß einem der vorhergehenden Ansprüche, **dadurch gekennzeichnet, dass** es darin besteht, alle Chromatographieschritte beim gleichen pH-Wert auszuführen.

13. Verfahren gemäß einem der vorhergehenden Ansprüche, **dadurch gekennzeichnet, dass** es sich um ein Online-Verfahren handelt.

14. Verfahren gemäß einem der vorhergehenden Ansprüche, **dadurch gekennzeichnet, dass** es bei Raumtemperatur durchgeführt wird.

15. Verfahren gemäß einem der vorhergehenden Ansprüche, **dadurch gekennzeichnet, dass** es unter sterilen Bedingungen durchgeführt wird.

## Claims

1. Method for purifying viruses obtained from a cell culture, which consists in separating by ion-exchange chromatography the viruses from the cell proteins and DNA originating from the culture, **characterized in that** it comprises at least one step of anion-exchange chromatography and one step of cation-exchange chromatography.

2. Method according to Claim 1, **characterized in that** the anions are strong anions.

3. Method according to either of the preceding claims, **characterized in that** the cations are strong cations.

4. Method according to one of the preceding claims, **characterized in that** the cation exchange is carried out after the anion exchange.

5. Method according to one of the preceding claims, **characterized in that** it also comprises a step of affinity chromatography by metal chelation.

6. Method according to the preceding claim, **characterized in that** the metal chelation is carried out using calcium ions.

7. Method according to one of the preceding claims, **characterized in that** the viruses to be purified are obtained by culturing on continuous and heteroploid cell lines.

8. Method according to one of the preceding claims, **characterized in that** the viruses to be purified are obtained by culturing on Vero cells.

9. Method according to one of the preceding claims, **characterized in that** the viruses to be purified are rabies viruses.

10. Method according to one of Claims 1 to 9, **characterized in that** the viruses to be purified are Japanese encephalitis viruses.

11. Method according to one of Claims 1 to 9, **characterized in that** the viruses to be purified are influenza viruses.

12. Method according to one of the preceding claims, **characterized in that** it consists in carrying out all of the chromatography steps at the same pH value.

13. Method according to one of the preceding claims, **characterized in that** it is an in-line method.

14. Method according to one of the preceding claims, **characterized in that** it is carried out at room temperature.

15. Method according to one of the preceding claims, **characterized in that** it is carried out under sterile conditions.
